(19) Europäisches Patentamt
European Patent Office
Office européen des brevets

(11) Veröffentlichungsnummer: **0 384 279 B1**

(12) # EUROPÄISCHE PATENTSCHRIFT

(45) Veröffentlichungstag der Patentschrift: **13.04.94**

(51) Int. Cl.[5]: **C07C 311/21**, C07C 311/29, A61K 31/18

(21) Anmeldenummer: **90102859.7**

(22) Anmeldetag: **14.02.90**

(54) **Benzolsulfonamide und Verfahren zu ihrer Herstellung.**

(30) Priorität: **18.02.89 DE 3905075**

(43) Veröffentlichungstag der Anmeldung:
**29.08.90 Patentblatt 90/35**

(45) Bekanntmachung des Hinweises auf die Patenterteilung:
**13.04.94 Patentblatt 94/15**

(84) Benannte Vertragsstaaten:
**AT BE CH DE DK ES FR GB GR IT LI LU NL SE**

(56) Entgegenhaltungen:
**EP-A- 0 288 028**
**DE-A- 2 617 180**

**CHEMICAL ABSTRACTS, Band 93, Nr. 23, 8. Dezember 1980, S. 484, Spalte 1, Zusammenfassung Nr. 220269e, Columbus, Ohio, USA; K. S. BURMISTROV et al: "Nature of the effect of the substitutent on the nitrogen atom on redox potentials of p-benzoquinone monoimines"**

**CHEMICAL ABSTRACTS, Band 75, Nr. 17, 25. Okfober 1971, S. 231, Spalte 2, Zusammenfassung Nr. 107790h, Columbus, Ohio, USA; N. K. STATSEK et al: "Toxicology of new polymer stabilizers such as derivatives of 4-hydroxydiphenylamine, arenesulfonic derivates of aminophenols, amino(arylamino)triazines, and alkylated salicyclic acids"**

(73) Patentinhaber: **HOECHST AKTIENGESELLSCHAFT**

**D-65926 Frankfurt(DE)**

(72) Erfinder: **Lang, Hans-Jochen, Dr.**
**Rüdesheimer Strasse 7**
**D-6238 Hofheim am Taunus(DE)**
Erfinder: **Granzer, Ernold, Dr. Dr**
**Falkensteiner Strasse 24**
**D-6233 Kelkheim/Taunus(DE)**
Erfinder: **Kerekjarto, Bela, Dr.**
**Weilbächer Wälder FA8**
**D-6238 Hofheim am Taunus(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft Benzolsulfonamide.

Es ist bekannt, daß Benzolsulfonamide der Formel IV

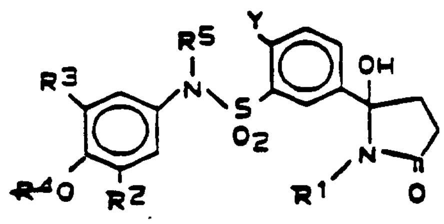

IV

salidiuretische und blutdrucksenkende Eigenschaften besitzen und gleichzeitig die atherogenen Lipide der LDL-Fraktion im Serum zu senken vermögen (Deutsche Offenlegungsschrift 37 13 757 entsprechend EP-A-0 288 028 und US Patent Nr. 4 849 444).

Für Hyperlipidämiker, die nicht gleichzeitig an Hypertonie und/oder Herzinsuffizienz erkrankt sind, erweist sich die Salidiurese und die Blutdrucksenkung als störend.

Der Erfindung liegt die Aufgabe zugrunde, Benzolsulfonamidderivate zur Verfügung zu stellen, die weder salidiuretische noch blutdrucksenkende Wirkung zeigen.

Es wurde gefunden, daß die salidiuretischen und blutdrucksenkenden Eigenschaften bei Benzolsulfonamiden der Formel I

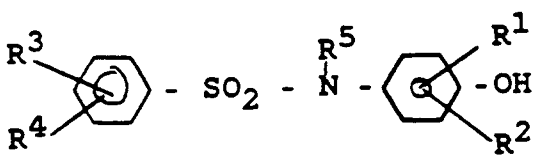

I

nicht mehr vorhanden sind.

Sulfonamidderivate von gehinderten Phenolen sind bereits beschrieben worden (vgl. DE-A-26 17 180 und Chem. Abstr. Vol. 75, 231, 107790 h (1971)). Entsprechend dem Stand der Technik werden sie als Stabilisatoren für Polymere verwendet.

Die Erfindung betrifft daher Benzolsulfonamide der Formel I, worin

- $R^1$ Methyl, Isopropyl, tert,-Butyl
- $R^2$ Isopropyl, tert.-Butyl
- $R^3$ Wasserstoff, Chlor, geradkettiges oder verzweigtes Alkoxy mit 1 bis 3 C-Atomen
- $R^4$ Trifluormethyl, einen Rest $-SO_n-A$, worin A Alkyl mit 1 bis 3 C-Atomen oder wenn $n = 2$, $NR^6R^7$ bedeutet mit $R^6$ und $R^7$ in der Bedeutung von Wasserstoff oder Alkyl mit 1 bis 4 C-Atomen, $n = 0$, 1, 2,
- $R^5$ Wasserstoff oder Alkyl mit 1-3 C-Atomen bedeuten.

Die Alkylreste in den Substituenten $R^4$ und $R^5$ sowie $R^6$ und $R^7$ sind geradkettig oder verzweigt.

Die Erfindung betrifft insbesondere die Verbindungen der Formel I zur Anwendung als Arzneimittel.

Besonders bevorzugt sind die folgenden Verbindungen der Formel I:

5-N-(3,5-Di-tert.-butyl-4-hydroxyphenylsulfamoyl)-2-chlorbenzol-N,N-dimethylsulfonamid
5-N-(3,5-Di-tert.-butyl-4-hydroxyphenylsulfamoyl)-2-chlorbenzol-N-methylsulfonamid
5-N-(3,5-Di-tert.-butyl-4-hydroxyphenylsulfamoyl)-2-chlorbenzolsulfonamid
3-N-(3,5-Di-tert.-butyl-4-hydroxyphenylsulfamoyl)-4-chlorbenzolsulfonamid.

Die Erfindung betrifft weiterhin ein Verfahren zur Herstellung der Verbindungen der Formel I, das dadurch gekennzeichnet ist, daß man Verbindungen der Formel II

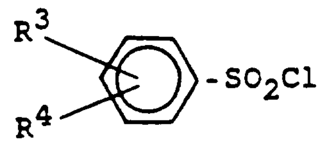

II

mit einem Amin der Formel III

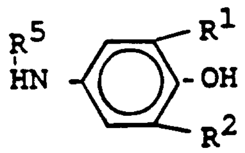

III,

worin $R^1$ bis $R^5$ die angegebene Bedeutung besitzen, umsetzt und gegebenenfalls die erhaltenen Verbindungen der Formel I, worin $R^5$ Wasserstoff bedeutet, durch übliche Alkylierung in Verbindungen der Formel I überführt, in denen $R^5$ einen Alkylrest mit 1 - 3 C-Atomen bedeutet.

Die Verbindungen der Formel II werden in an sich bekannter Weise mit einem Aminophenol der Struktur III, worin $R_1$ bis $R_5$ die angegebene Bedeutung besitzen, unter Bildung der Verbindungen I zur Reaktion gebracht. Dabei verfährt man bevorzugt so, daß man beide Reaktanten in einem Molverhältnis von 1:1 in Wasser oder einem inerten polaren organischen Lösungsmittel, wie Dimethylformamid, Dimethylacetamid, Dioxan, Tetrahydrofuran, Diethylenglycoldimethylether, in niederen Alkoholen mit 1-4 C-Atomen, z.B. in Methanol, Ethanol, Isopropanol, ebenso auch in einem Alkansäureniederalkylester, beispielweise Essigsäuremethyl- oder -ethylester reagieren läßt.

Vorteilhaft erweist sich die Anwesenheit von mindestens 1, besser von 2 oder mehr Molen, einer geeigneten protonenabfangenden Hilfsbase, wie beispielsweise von Alkali- oder Erdalkalihydroxid wie KOH, NaOH, $Ca(OH)_2$, Alkalimetallcarbonat oder -hydrogencarbonat wie $Na_2CO_3$, $K_2CO_3$, $NaHCO_3$, $KHCO_3$ oder vorzugsweise von einem tertiären Amin wie Trimethylamin, Triethylamin, Tripropylamin, Tributylamin, Dicyclohexylethylamin oder Pyridin, aber auch von Alkali- sowie Erdalkalisalzen schwacher Alkancarbonsäuren wie z.B. Natriumacetat.

Die Sulfonsäurechloride der Formel II und die Aminophenole der Formel III, worin $R^1$ bis $R^5$ die angegebene Bedeutung haben sind weitgehend literaturbekannt und können falls nicht bekannt in Analogie zu den bekannten Verfahren gewonnen werden.

Die Verfahrensprodukte sind wertvolle Arzneimittel und zeichnen sich durch sehr günstige therapeutisch verwertbare Eigenschaften aus.

Die Erfindung betrifft daher auch pharmazeutische Präparate auf Basis der Verbindungen der Formel I, sowie ihre Verwendung als Arzneimittel, insbesondere zur Behandlung von Lipidstoffwechselstörungen. Die Erfindung betrifft ferner die Verwendung von Verbindungen der Formel I zur Herstellung von Arzneimitteln.

Erhöhte VLDL- und LDL-Spiegel sind nach heutiger Ansicht maßgeblich an der Entstehung und am Fortschreiten der Atherosklerose beteiligt. Insbesondere ist oxidiertes LDL stark atherogen und cytotoxisch. Die Senkung erhöhter VLDL- und LDL-Spiegel und die Verhinderung pathophysiologischer Oxidationsvorgänge zur Beeinflussung atherosklerotischer Prozesse ist daher von großem therapeutischen Interesse. Bei einer Reihe von Patienten spielt ein erhöhter Blutdruck zusätzlich eine wichtige Rolle. Für diese Patienten wurden Benzolsulfonamide beschrieben (DE-A-37 13 757), die salidiuretische und blutdrucksenkende Eigenschaften besitzen und gleichzeitg die atherogenen Lipide der LDL-Fraktion im Serum zu senken vermögen. Für Patienten, die aber nicht an Hypertonie und/oder Herinsuffizienz erkrankt sind aber erhöhte Lipidspiegel haben, ist die Salidiurese und Blutdrucksenkung störend. Die erfindungsgemäßen Sulfonamide der Formel I zeichnen sich durch ihre neue Wirkqualität aus, da salidiuretische und blutdrucksenkende Eigenschaften nicht mehr vorhanden sind. Die neuen Verbindungen vermögen in wesentlich stärkerem Maße die atherogenen Lipide zu senken und können oxidative Prozesse verhindern. Sie erfüllen daher die Anforderungen als Arzneimittel zur Regulation der Blutfette für Hyperlipidämiker, die keinen Bluthochdruck haben und nicht unter Herzinsuffizienz leiden. Darüber hinaus können die Substanzen wertvolle Arzneimittel sein bei der Behandlung von Krankheiten, bei denen oxidative Prozesse eine Rolle spielen. Von großer Bedeutung ist die Tatsache, daß diese Wirkqualität mit Verbindungen aus der Klasse der Benzolsulfonamide erzielt werden konnte, die nicht zuletzt wegen ihrer guten Verträglichkeit zu den Basistherapeutika gehört.

Aus zahlreichen Publikationen der letzten Jahre geht hervor, daß der Entstehung atherosklerotischer Plaques Verletzungen des Gefäßendothels vorausgehen. Derartige Endothelverletzungen können durch Fettsäureperoxide und Radikalreaktionen aktiver Sauerstoffspezies verursacht werden, so daß mit einer Verminderung dieser Lipidperoxide, die im wesentlichen in der LDL-Fraktion transportiert werden, zwangsläufig eine Verminderung des atherogenen Risikos verbunden ist.

Es konnte nun gezeigt werden, daß die erfindungsgemäßen Verbindungen bereits im Bereich 1-10 mg/kg an der Ratte die LDL-Fraktion zu senken vermögen ohne Beeinflussung der HDL-Fraktion.
Darüber hinaus hemmt die erfindungsgemäßen Verbindungen die Lipidperoxidation, die als Maß für die Bildung von atherogenem oxidierten LDL angesehen werden kann (Hemmung der NADPH-abhängigen mikrosomalen Lipidperoxidation, Molondialdehydbildung $IC_{50} \leqq 1\ \mu M$).

Die Verbindungen I werden in Dosen von mindestens 0,5 mg/kg und Tag, vorzugsweise 1,0 mg/kg/Tag, bis 15 mg/kg/Tag, vorzugsweise 10 mg/kg/Tag, angewendet, bezogen auf einen erwachsenen Menschen von etwa 75 kg Körpergewicht.

Als therapeutische Zubereitung der neuen Verbindungen kommen vor allem Tabletten, Dragees, Kapseln, Säfte und Suppositiorien sowie auch Ampullen zur parenteralen Verabreichung (i.v., s.c., und i.m.) in Frage. Die Verbindungen der Formel I können dabei entweder allein oder mit pharmakologisch annehmbaren Trägern vermischt, angewandt werden. Eine orale Anwendungsform wird bevorzugt. Zu diesem Zweck werden die aktiven Verbindungen vorzugsweise mit an sich bekannten Substanzen vermischt und durch an sich bekannte Methoden in geeignete Darreichungsformen gebracht, wie Tabletten, Steckkapseln, wäßrige oder ölige Suspensionen oder wäßrige oder ölige Lösungen. Als inerte Träger können z. B. Magnsiumkarbonat, Milchzucker oder Maisstärke unter Zusatz anderer Stoffe wie z. B. Magnesiumstearat verwendet werden. Dabei kann die Zubereitung als Trocken- oder Fechtgranulat erfolgen. Als ölige Trägerstoffe oder Lösungsmittel kommen besonders pflanzliche und tierische Öle in Betracht, wie z. B. Sonnenblumenöl oder Lebertran.

Die Zubereitungen können bei der Behandlung von Lipidstoffwechselstörungen außer den üblichen Füll- und Trägerstoffen noch ein Antihypertensivum, wie beispielsweise ein Saluretikum, Reserpin, Hydralazin, Guanethidin, $\alpha$-Methyldopa, Clonidin oder ein $\beta$-Sympatholytikum, oder ein antihyperurikämisch wirksames Mittel, ein orales Antidiabetikum, ein Geriatrikum oder ein Mittel mit durchblutungssteigernder Wirkung enthalten.

In den nachfolgenden Beispielen sind die Schmelz- und Zersetzungspunkte der Ausführungsbeispiele nicht korrigiert.

Beispiele 1 - 32

Allgemeine Vorschrift zur Herstellung der Verbindungen der Formel I:

Zu einer Lösung von 0,1 Mol Sulfonsäurechlorid II und 0,3 Mol Triethylamin in 500 ml Essigsäureethylester fügt man portionsweise eine Suspension oder Lösung von 0,1 bis 0,2 Mol Aminophenol III in 450 ml Essigester. Die Temperatur wird zwischen 30 und 40 °C gehalten. Man rührt 3 Stunden bei 50 °C, kühlt ab, versetzt mit Wasser, trennt die organische Phase ab und extrahiert die wäßrige Phase mehrmals mit Essigester. Die vereinigten organischen Phasen werden mit Wasser gewaschen, über Natriumsulfat getrocknet, und das Lösungsmittel wird abdestilliert. Weitere Reinigung durch Kristallisation.

**Tabelle 1**

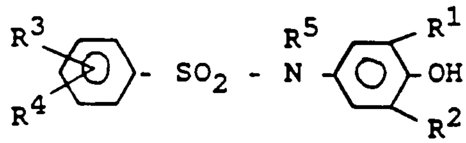

I

| Beispiel | $R^1$ | $R^2$ | $R^4$ | $R^3$ | $R^5$ | Fp. |
|---|---|---|---|---|---|---|
| 1 | t-But | t-But | 3-$H_2NO_2S$- | 4-Cl | H | 217 °C |
| 2 | i-Prop | i-Prop | 3-$H_2NO_2S$- | 4-Cl | H | 210 °C |
| 3 | t-But | t-But | 3-$H_2NO_2S$- | 4-Cl/6-$NH_2$ | H | 238 °C |
| 4 | t-But | t-But | 4-$H_2NO_2S$- | H | H | 191 °C |
| 5 | t-But | t-But | 3-$H_2NO_2S$- | H | H | 225 °C |
| 6 | t-But | t-But | 3-$MeNHO_2S$- | 4-Cl | H | 179 °C |
| 7 | t-But | t-But | 3-$Me_2NO_2S$- | 4-Cl | H | 170 °C |
| 8 | t-But | t-But | 3-$H_2NO_2S$- | 4-Me | H | 166-168 °C |
| 9 | t-But | t-But | 3-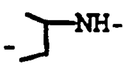NH-$SO_2$- | 4-Cl | H | 191 °C |
| 10 | t-But | t-But | 5-$H_2NO_2S$ | 2-Cl | H | 201 °C |
| 11 | t-But | t-But | 3-MeS- | H | H | 158-160 °C |
| 12 | t-But | t-But | 4-Me-S- | H | H | 193-195 °C |
| 13 | t-But | t-But | 3-Me-SO- | H | H | 187 °C |
| 14 | t-But | t-But | 3-Me-$SO_2$- | H | H | 177-178 °C |
| 15 | t-But | t-But | 4-MeSO- | H | H | 219 °C |
| 16 | t-But | t-But | 2Me-S- | H | H | 135 °C |
| 17 | t-But | t-But | 2-Me-$SO_2$ | H | H | 223 °C |
| 18 | t-But | t-But | 4-$CF_3$- | H | H | 182-183 °C |
| 19 | t-But | t-But | 3-$CF_3$ | 4-Cl | H | 135 °C |
| 20 | t-But | t-But | 3-$CF_3$ | H | H | 144-145 °C |
| 21 | t-But | t-But | 2-$CF_3$ | H | H | 129-131 °C |
| 22 | t-But | t-But | 5-$CF_3$ | 2-Cl | H | 192 °C |
| 23 | i-Prop | i-Prop | 3-$CF_3$ | 4-Cl | H | 178 °C |
| 24 | i-Prop | i-Prop | 3-$MeSO_2$- | H | H | 159 °C |

**Forsetzung von Tabelle 1**

| Beispiel | $R^1$ | $R^2$ | $R^4$ | $R^3$ | $R^5$ | Fp. |
|---|---|---|---|---|---|---|
| 25 | Me | t-But | 3-$CF_3$ | H | H | 134-135 °C |
| 26 | Me | t-But | 4-$CF_3$ | H | H | 162 °C |
| 27 | t-But | t-But | 3-Et-$\overset{H}{N}$-$SO_2$- | 4-Cl | H | 164 °C |
| 28 | t-But | t-But | 3-iProp$\overset{H}{N}$-$SO_2$- | 4-Cl | H | 187 °C |
| 29 | t-But | t-But | 4-$H_2NO_2S$ | 3-Cl | H | 185 °C |
| 30 | t-But | t-But | 5-$Me_2NO_2S$- | 2Cl | H | 215 °C |
| 31 | t-But | t-But | 3-$H_2N$-$O_2S$- | 4-MeO- | H | 214-215 °C |
| 32 | t-But | t-But | 3-$Me_2NO_2S$- | 4-Cl | H | 321-134 °C |

## Patentansprüche

**Patentansprüche für folgende Vertragsstaaten : AT, BE, CH, DE, DK, FR, GB, IT, LI, LU, NL, SE**

**1.** Benzolsulfonamide der Formel I

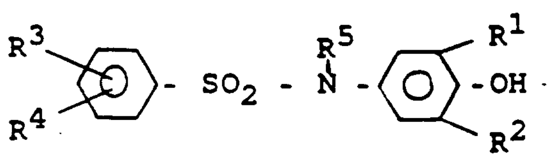

I

worin

- $R^1$ Methyl, Isopropyl, tert,-Butyl
- $R^2$ Isopropyl, tert.-Butyl
- $R^3$ Wasserstoff, Chlor, geradkettiges oder verzweigtes Alkoxy mit 1 bis 3 C-Atomen
- $R^4$ Trifluormethyl, einen Rest -$SO_n$-A, worin A Alkyl mit 1 bis 3 C-Atomen oder wenn $n = 2$, $NR^6R^7$ bedeutet mit $R^6$ und $R^7$ in der Bedeutung von Wasserstoff oder Alkyl mit 1 bis 4 C-Atomen, $n = 0, 1, 2$,
- $R^5$ Wasserstoff oder Alkyl mit 1-3 C-Atomen bedeuten.

**2.** Verbindung I nach Anspruch 1, dadurch gekennzeichnet, daß sie aus der Gruppe
5-N-(3,5-Di-tert.-butyl-4-hydroxyphenylsulfamoyl)-2-chlorbenzol-N,N-dimethylsulfonamid
5-N-(3,5-Di-tert.-butyl-4-hydroxyphenylsulfamoyl)-2-chlorbenzol-N-methylsulfonamid
5-N-(3,5-Di-tert.-butyl-4-hydroxyphenylsulfamoyl)-2-chlorbenzolsulfonamid
3-N-(3,5-Di-tert.-butyl-4-hydroxyphenylsulfamoyl)-4-chlorbenzolsulfonamid
ausgewählt ist.

**3.** Verfahren zur Herstellung der Verbindungen I nach Anspruch 1, dadurch gekennzeichnet, daß man Verbindungen der Formel II

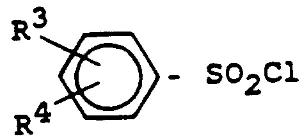

II

mit Verbindungen der Formel III

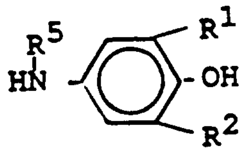

III,

wobei $R^1$ bis $R^5$ die angegebene Bedeutung besitzen, umsetzt und gegebenenfalls eine erhaltene Verbindung der Formel I, worin $R^5$ Wasserstoff bedeutet, alkyliert zu Verbindungen der Formel I mit $R^5$ gleich Alkyl mit 1 - 3 C-Atomen.

**4.** Verbindungen der Formel I gemäß Anspruch 1 zur Anwendung als Arzneimittel.

**5.** Verwendung von Verbindungen der Formel I gemäß Anspruch 1 zur Herstellung eines Arzneimittels.

**6.** Verbindung I nach Anspruch 1 zur Anwendung als Hypolipidämikum.

**7.** Verwendung einer Verbindung I nach Anspruch 1 zur Herstellung eines Medikaments mit lipidsenkender Wirkung und/oder antioxidativen Eigenschaften.

**8.** Pharmazeutische Zubereitung enthaltend eine wirksame Menge einer Verbindung I nach Anspruch 1 und pharmazeutisch üblichen Zusatzstoffen.

**Patentansprüche für folgende Vertragsstaaten : ES, GR**

**1.** Verfahren zur Herstellung von Benzolsulfonamiden der Formel I

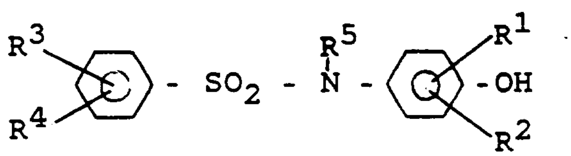

I

worin

$R^1$ Methyl, Isopropyl, tert,-Butyl

$R^2$ Isopropyl, tert.-Butyl

$R^3$ Wasserstoff, Chlor, geradkettiges oder verzweigtes Alkoxy mit 1 bis 3 C-Atomen

$R^4$ Trifluormethyl, einen Rest $-SO_n-A$, worin A Alkyl mit 1 bis 3 C-Atomen oder wenn $n = 2$, $NR^6R^7$ bedeutet mit $R^6$ und $R^7$ in der Bedeutung von Wasserstoff oder Alkyl mit 1 bis 4 C-Atomen, $n = 0, 1, 2$,

$R^5$ Wasserstoff oder Alkyl mit 1-3 C-Atomen bedeuten,

dadurch gekennzeichnet, daß man Verbindungen der Formel II

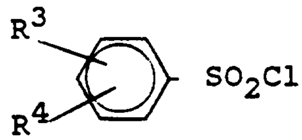

II

mit Verbindungen der Formel III

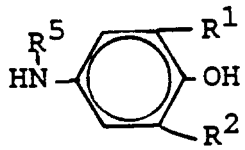

III,

wobei $R^1$ bis $R^5$ die angegebene Bedeutung besitzen, umsetzt und gegebenenfalls eine erhaltene Verbindung der Formel I, worin $R^5$ Wasserstoff bedeutet, alkyliert zu Verbindungen der Formel I mit $R^5$ gleich Alkyl mit 1 - 3 C-Atomen.

**2.** Verfahren nach Anspruch 1, dadurch gekennzeichnet, daß man eine Verbindung ausgewählt aus der Gruppe
5-N-(3,5-Di-tert.-butyl-4-hydroxyphenylsulfamoyl)-2-chlorbenzol-N,N-dimethylsulfonamid
5-N-(3,5-Di-tert.-butyl-4-hydroxyphenylsulfamoyl)-2-chlorbenzol-N-methylsulfonamid
5-N-(3,5-Di-tert.-butyl-4-hydroxyphenylsulfamoyl)-2-chlorbenzolsulfonamid und
3-N-(3,5-Di-tert.-butyl-4-hydroxyphenylsulfamoyl)-4-chlorbenzolsulfonamid
herstellt.

**3.** Verwendung von Verbindungen der Formel I gemäß Anspruch 1 zur Herstellung eines Arzneimittels.

**4.** Verwendung einer Verbindung I nach Anspruch 1 zur Herstellung eines Medikaments mit lipidsenkender Wirkung und/oder antioxidativen Eigenschaften.

**5.** Verfahren zur Herstellung einer pharmazeutischen Zubereitung, dadurch gekennzeichnet, daß man eine wirksame Menge einer Verbindung I erhältlich nach Anspruch 1 und pharmazeutisch üblichen Zusatzstoffen in eine geeignete Darreichungsform überführt.

**Claims**
**Claims for the following Contracting States : AT, BE, CH, DE DK, FR, GB, IT, LI, LU, NL, SE**

**1.** A benzenesulfonamide of the formula I

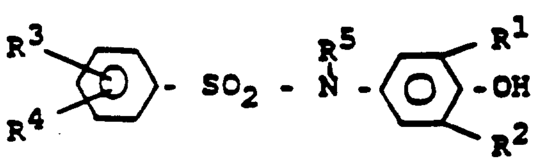

I

in which

- $R^1$ denotes methyl, isopropyl or tert.-butyl,
- $R^2$ denotes isopropyl or tert.-butyl,
- $R^3$ denotes hydrogen, chlorine, or straight-chain or branched alkoxy having 1 to 3 carbon atoms,
- $R^4$ denotes trifluoromethyl or a radical $-SO_n-A$, in which A denotes alkyl having 1 to 3 carbon atoms or, if $n=2$, denotes $NR^6R^7$ with $R^6$ and $R^7$ denoting hydrogen or alkyl having 1 to 4 carbon atoms, $n=0$, 1 or 2, and
- $R^5$ denotes hydrogen or alkyl having 1 - 3 carbon atoms.

2. A compound I as claimed in claim 1, which is selected from the group comprising
5-N-(3,5-di-tert.-butyl-4-hydroxyphenylsulfamoyl)-2-chlorobenzene-N,N-dimethylsulfonamide
5-N-(3,5-di-tert.-butyl-4-hydroxyphenylsulfamoyl)-2-chlorobenzene-N-methylsulfonamide
5-N-(3,5-di-tert.-butyl-4-hydroxyphenylsulfamoyl)-2-chlorobenzenesulfonamide
3-N-(3,5-di-tert.-butyl-4-hydroxyphenylsulfamoyl)-4-chlorobenzenesulfonamide.

3. A process for the preparation of the compounds I as claimed in claim 1, which comprises reacting compounds of the formula II

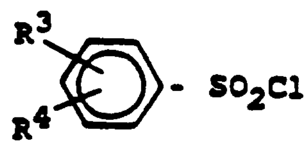

II

with compounds of the formula III

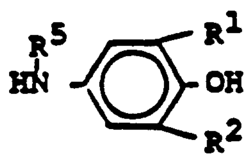

III,

where $R^1$ to $R^5$ have the meaning indicated and, if appropriate, alkylating a resulting compound of the formula I in which $R^5$ denotes hydrogen to give compounds of the formula I with $R^5$ equal to alkyl with 1 - 3 carbon atoms.

4. A compound of the formula I as claimed in claim 1 for use as a pharmaceutical.

5. The use of compounds of the formula I as claimed in claim 1 for the production of a pharmaceutical.

6. A compound I as claimed in claim 1 for use as a hypolipidemic.

7. The use of a compound I as claimed in claim 1 for the production of a medicament having lipid-lowering action and/or antioxidative properties.

8. A pharmaceutical preparation containing an effective amount of a compound I as claimed in claim 1 and pharmaceutically customary additives.

**Claims for the following Contracting States : ES, GR**

1. A process for the preparation of benzenesulfonamides of the formula I

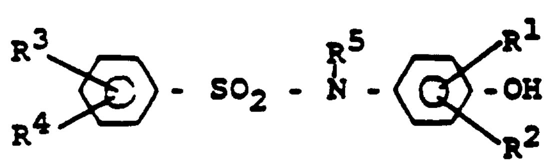

I

in which
- $R^1$ denotes methyl, isopropyl or tert.-butyl,
- $R^2$ denotes isopropyl or tert.-butyl,
- $R^3$ denotes hydrogen, chlorine, or straight-chain or branched alkoxy having 1 to 3 carbon atoms,
- $R^4$ denotes trifluoromethyl or a radical $-SO_n-A$, in which A denotes alkyl having 1 to 3 carbon atoms or, if n = 2, denotes $NR^6R^7$ with $R^6$ and $R^7$ denoting hydrogen or alkyl having 1 to 4

carbon atoms, n = 0, 1 or 2, and

$R^5$ denotes hydrogen or alkyl having 1 - 3 carbon atoms, which comprises reacting compounds of the formula II

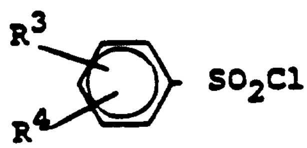

II

with compounds of the formula III

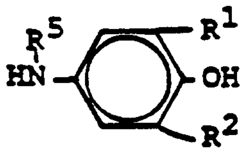

III,

where $R^1$ to $R^5$ have the meaning indicated and, if appropriate, alkylating a resulting compound of the formula I in which $R^5$ denotes hydrogen to give compounds of the formula I with $R^5$ equal to alkyl with 1 - 3 carbon atoms.

**2.** The process as claimed in claim 1, which comprises preparing a compound selected from the group comprising
5-N-(3,5-di-tert.-butyl-4-hydroxyphenylsulfamoyl)-2-chlorobenzene-N,N-dimethylsulfonamide
5-N-(3,5-di-tert.-butyl-4-hydroxyphenylsulfamoyl)-2-chlorobenzene-N-methylsulfonamide
5-N-(3,5-di-tert.-butyl-4-hydroxyphenylsulfamoyl)-2-chlorobenzenesulfonamide and
3-N-(3,5-di-tert.-butyl-4-hydroxyphenylsulfamoyl)-4-chlorobenzenesulfonamide.

**3.** The use of compounds of the formula I as in claim 1 for the production of a pharmaceutical.

**4.** The use of a compound I as in claim 1 for the production of a medicament having lipid-lowering action and/or antioxidative properties.

**5.** A process for preparing a pharmaceutical preparation, which comprises transforming an effective amount of a compound I obtainable as claimed in claim 1 and pharmaceutically customary additives into a suitable administration form.

**Revendications**

**Revendications pour les Etats contractants suivants : AT, BE, CH, DE, DK, FR, GB, IT, LI, LU, NL, SE**

**1.** Benzènesulfonamides de formule I

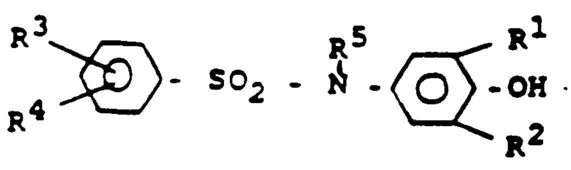

I

dans laquelle

$R^1$ représente le groupe méthyle, isopropyle, tert-butyle,

$R^2$ représente le groupe isopropyle, tert-butyle,

$R^3$ représente un atome d'hydrogène ou de chlore ou un groupe alcoxy à chaîne droite ou ramifiée ayant de 1 à 3 atomes de carbone,

$R^4$ représente le groupe trifluorométhyle ou un radical $-SO_n-A$, dans lequel A représente un

groupe alkyle ayant de 1 à 3 atomes de carbone, ou, lorsque n = 2, $NR^6R^7$, $R^6$ et $R^7$ représentant un atome d'hydrogène ou un groupe alkyle ayant de 1 à 4 atomes de carbone, n = 0, 1 ou 2,

$R^5$ représente un atome d'hydrogène ou un groupe alkyle ayant de 1 à 3 atomes de carbone.

**2.** Composé I selon la revendication 1, caractérisé en ce qu'il est choisi parmi les composés suivants
5-N-(3,5-di-tert-butyl-4-hydroxyphénylsulfamoyl)-2-chlorobenzène-N,N-diméthylsulfonamide,
5-N-(3,5-di-tert-butyl-4-hydroxyphénylsulfamoyl)-2-chlorobenzène-N-méthylsulfonamide,
5-N-(3,5-di-tert-butyl-4-hydroxyphénylsulfamoyl)-2-chlorobenzènesulfonamide,
3-N-(3,5-di-tert-butyl-4-hydroxyphénylsulfamoyl)-4-chlorobenzènesulfonamide.

**3.** Procédé pour la préparation des composés I selon la revendication 1, caractérisé en ce que l'on fait réagir des composés de formule II

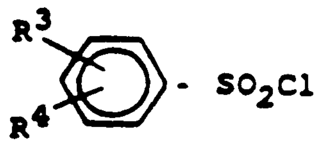

II

avec des composés de formule III

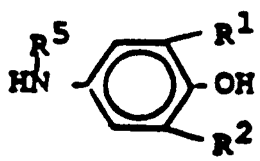

III

formules dans lesquelles $R^1$ á $R^5$ ont les significations indiquées, et éventuellement on soumet à une alkylation un composé de formule I obtenu, dans lequel $R^5$ représente un atome d'hydrogène, pour aboutir à des composés de formule I dans lesquels $R^5$ est un groupe alkyle ayant de 1 à 3 atomes de carbone.

**4.** Composés de formule I selon la revendication 1, pour utilisation en tant que médicament.

**5.** Utilisation des composés de formule I selon la revendication 1, pour la fabrication d'un médicament.

**6.** Composés I selon la revendication 1, pour utilisation en tant qu'hypolipidémiant.

**7.** Utilisation d'un composé I selon la revendication 1, pour la fabrication d'un médicament à effet hypolipidémiant et/ou à propriétés antioxydantes.

**8.** Composition pharmaceutique contenant une quantité efficace d'un composé I selon la revendication 1 et des additifs pharmaceutiquement usuels.

**Revendications pour les Etats contractants suivants : ES, GR**

**1.** Procédé pour la préparation de Benzènesulfonamides de formule I

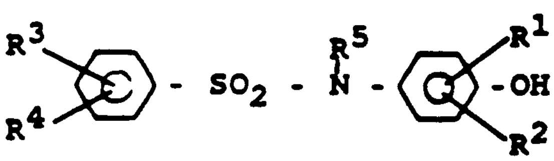

I

dans laquelle

$R^1$ représente le groupe méthyle, isopropyle, tert-butyle,

$R^2$ représente le groupe isopropyle, tert-butyle,

$R^3$ représente un atome d'hydrogène ou de chlore ou un groupe alcoxy à chaîne droite ou ramifiée ayant de 1 à 3 atomes de carbone,

$R^4$ représente le groupe trifluorométhyle ou un radical $-SO_n-A$, dans lequel A représente un groupe alkyle ayant de 1 à 3 atomes de carbone, ou, lorsque n = 2, $NR^6R^7$, $R^6$ et $R^7$ représentant un atome d'hydrogène ou un groupe alkyle ayant de 1 à 4 atomes de carbone, n = 0, 1 ou 2,

$R^5$ représente un atome d'hydrogène ou un groupe alkyle ayant de 1 à 3 atomes de carbone,

caractérisé en ce que l'on fait réagir des composés de formule II

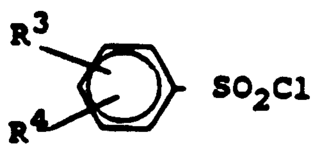

II

avec des composés de formule III

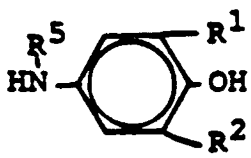

III

formules dans lesquelles $R^1$ á $R^5$ ont les significations indiquées, et éventuellement on soumet à une alkylation un composé de formule I obtenu, dans lequel $R^5$ représente un atome d'hydrogène, pour aboutir à des composés de formule I dans lesquels $R^5$ est un groupe alkyle ayant de 1 à 3 atomes de carbone.

**2.** Procédé selon la revendication 1, caractérisé en ce que l'on prépare un composé choisi parmi les composés suivants
5-N-(3,5-di-tert-butyl-4-hydroxyphénylsulfamoyl)-2-chlorobenzène-N,N-diméthylsulfonamide,
5-N-(3,5-di-tert-butyl-4-hydroxyphénylsulfamoyl)-2-chlorobenzène-N-méthylsulfonamide,
5-N-(3,5-di-tert-butyl-4-hydroxyphénylsulfamoyl)-2-chlorobenzènesulfonamide,
3-N-(3,5-di-tert-butyl-4-hydroxyphénylsulfamoyl)-4-chlorobenzènesulfonamide.

**3.** Utilisation de composés de formule I selon la revendication 1, pour la fabrication d'un médicament.

**4.** Utilisation d'un composé I selon la revendication 1, pour la fabrication d'un médicament à activité hypolipidémiante et/ou à propriétés antioxydantes.

**5.** Procédé pour la préparation d'une composition pharmaceutique, caractérisé en ce que l'on met sous une forme d'administration appropriée une quantité efficace d'un composé I préparable selon la revendication 1 et des additifs pharmaceutiquement usuels.